# EUROPEAN PATENT SPECIFICATION

(11) **EP 3 012 756 B1**
(45) Date of publication and mention of the grant of the patent: **13.01.2021**
(21) Application number: 15190371.3
(22) Date of filing: 19.10.2015
(51) Int. Cl.: G16H 20/60, G16H 20/30

(54) **COMPUTING WEIGHT CONTROL PROFILE**
BERECHNUNG DES GEWICHTSKONTROLLPROFILS
PROFIL DE COMMANDE DE CALCUL DE POIDS

(30) Priority: 20.10.2014 US 201414518524
(43) Date of publication of application: 27.04.2016
(73) Proprietor: Polar Electro Oy, 90440 Kempele (FI)
(72) Inventor: Palatsi, Matti, 90440 Kempele (FI); Kampman, Ville, 90440 Kempele (FI); Thurlin, Tomi, 90440 Kempele (FI)
(74) Representative: Kolster Oy Ab

(56) References cited:
- EP-A1- 1 473 655
- US-A1- 2010 130 890
- US-A1- 2012 215 128
- US-A1- 2014 258 208
- US-B1- 6 811 516

## Description

### TECHNICAL FIELD

The invention relates to activity monitoring systems and information processing algorithms.

### TECHNICAL BACKGROUND

There are commercially available activity-monitoring systems configured to monitor daily activity of a user by employing motion sensors, heart activity sensors, etc. Such systems may be used to computer daily, weekly, or monthly energy expenditure of the user. Measured activity may be uploaded to a server computer accessible with a web browser, for example. The user may then evaluate long-term activity on the basis of records stored in his/her user account in the server computer

Prior art documents US 2012/215128 A1 (BARNETT JOHN THOMAS [US] ET AL) 23 August 2012 (2012-08-23) and EP 1 473 655 A1 (POLAR ELECTRO OY [FI]) 3 November 2004 (2004-11-03) disclose systems providing weight management instructions based on the current situation between the goal and the achieved progress.

### BRIEF DESCRIPTION

The invention is defined by the appended independent claims.

Some embodiments of the invention are defined in the dependent claims.

### BRIEF DESCRIPTION OF THE DRAWINGS

In the following the invention will be described in greater detail by means of preferred embodiments with reference to the accompanying drawings, in which
Figure 1 illustrates an activity monitoring system according to an embodiment of the invention;
Figures 2A to 2D illustrates some low diagrams, according to some embodiments of the invention;
Figure 3 illustrates an embodiment of a user interface of a portable electronic device according to an embodiment of the invention;
Figure 4 illustrates adjusting the way guidance is provided in reaching a set weight development target, according to an embodiment of the invention;
Figures 5A and 5B illustrate display views illustrating said adjustment instructions and effect of such adjustment instructions according to some embodiments of the invention;
Figure 6 illustrates a flow diagram of a process for computing activity adjustment instructions according to an embodiment of the invention;
Figure 7 illustrates a flow diagram of a process for computing nutrition intake adjustment instructions according to an embodiment of the invention;
Figure 8 illustrates a display view comprising a component enabling implementation of the activity adjustment instructions into activity targets according to an embodiment of the invention;
Figure 9 illustrates a flow diagram of a process for implementing the activity adjustment instructions into activity targets according to an embodiment of the invention; and
Figures 10 and 11 illustrate block diagrams of structures of apparatuses according to some embodiments of the invention.

### DETAILED DESCRIPTION OF EMBODIMENTS

The following embodiments are exemplary. Although the specification may refer to "an", "one", or "some" embodiment(s) in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may contain also features/structures that have not been specifically mentioned.

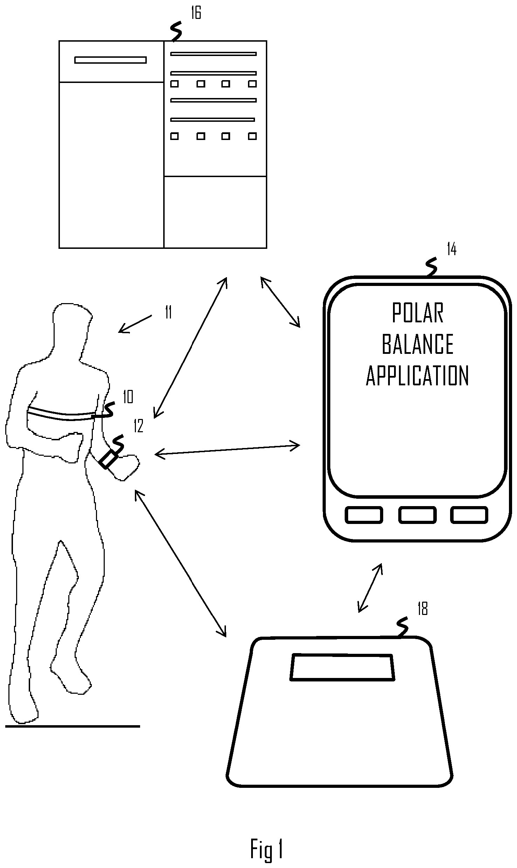

Figure 1 illustrates a physical training environment to which embodiments of the invention may be applied. Figure 1 illustrates an activity monitoring system comprising an activity monitoring apparatus 12 comprising a simplified user interface and a portable electronic device 14 comprising a user interface with more features. The activity monitoring system may further comprise one or more external sensor devices 10 such as a heart activity transmitter, a stride sensor, a positioning sensor, etc. Referring to Figure 1, a user 11 may carry an activity monitoring apparatus 12. The activity monitoring apparatus may be a portable or wearable device such as a wrist device 12 or another wearable training computer. The wrist device 12 may comprise at least one physical activity sensor configured to measure user's physical activity motion induced by the user 11 to the wrist device 12 by moving a hand in which the user 11 wears the wrist device 12.

In an embodiment, the physical activity sensor comprises a heart activity sensor configured to measure user's heart activity characterized by beat to beat interval, heart rate and/or heart rate variability.

In an embodiment, the physical activity sensor comprises a motion sensor configured to measure motion induced by the user 11 to the wrist device 12 by moving a hand in which the user 11 wears the wrist device 12.

In an embodiment, the motion sensor(s) comprise at least one of the following: an accelerometer, a magnetometer, and a gyroscope.

In an embodiment, the motion sensor comprises an accelerometer and a gyroscope. The motion sensor may further comprise sensor fusion software for combining the accelerometer data and gyroscope data so as to provide physical quantities, such as acceleration data, velocity data, or limb trajectory data in a reference coordinate system having orientation defined by a predetermined gyroscope orientation.

In an embodiment, the motion sensor comprises a gyroscope and a magnetometer. The motion sensor may further comprise sensor fusion software to combine gyroscope data and magnetometer data so as to provide a reference coordinate system for the gyroscope based on the Earth magnetic field measured by the magnetometer. In general, the sensor fusion software described above may combine measurement data acquired from at least two motion sensors such that measurement data acquired from one motion sensor is used to establish the reference coordinate system for the measurement data acquired from at least one other motion sensor.

The system may further comprise a weighing device 18 such as a scale configured to measure weight of a human, e.g. the user 11. The weighing device 18 may comprise a weighing unit based on a strain gauge or another circuitry converting a weight applied to the weighing device 18 by the user 11 or another entity being measured with the weighing device 18. The weighing unit may output a signal representing the measured weight to a communication circuitry configured to output the measured weight according to a determined communication protocol, e.g. a wireless or a wired communication protocol.

The system may further comprise a server computer 16 connected to at least one computer network, e.g. the Internet. The server computer 16 may store user accounts storing activity data of users, e.g. the user 11. Any one or all of the portable electronic device 14, the wrist device 12, and the weighing device 18 may be configured to establish an end-to-end communication connection with the server computer 16 and to upload and/or download data with the server computer 16. The portable electronic device 14, the wrist device 12, and the weighing device 18 may upload measurement data to the user's 11 user account stored server computer 16 and, in some embodiments, receive configuration data such as training targets from the server computer 16.

In an embodiment, the server computer 16 may store an identifier of a personal device associated with the user 11, e.g. the wrist computer 12 or the portable electronic device 14. The identifier may be a communication address of the device 12, 14 such as a medium access control (MAC) address or an internet protocol (IP) address. Each device 12, 14 may transmit its own identifier to the server computer together with the measurement data, and the server computer 16 may use the identifier to associate the measurement data with the user's 11 user account. In this manner, there is no need to log into the user account in order to store the data and, the data transfer may be carried out automatically without user intervention. In a similar manner, a similar identifier of the weighing device 18 may be registered to the user's 11 user account when the weighing device is associated with a single user. When a plurality of users share the same weighing device, the user currently using the weighing device may authenticate to the weighing device 18 and, as a consequence, the weighing device may associate the measured weight with a user identifier associated with the user and transmit the user identifier together with the measurement data to the server computer 16 such that the server computer may use the user identifier to find the user's user account and store the measurement data to the correct account.

The portable electronic device 14 may be a mobile phone, a smart phone, a palm device, a tablet computer, or a portable digital assistant. The portable electronic device 14 and the wrist device 12 may be configured to establish a wireless communication connection with one another and exchange activity data over the wireless communication connection. The wireless communication connection may be established according to Bluetooth® specifications, e.g. Bluetooth Low Energy. The activity monitoring system may employ the simplified user interface of the wrist device 12 to display coarse activity information and the sophisticated user interface of the portable electronic device to display the activity information in a higher display resolution.

In an embodiment, the portable electronic device 14 comprises a communication circuitry configured to establish the wireless communication connection with a communication circuitry of the wrist device 12, a display screen, and at least one processor configured to receive at least one of motion measurement data and heart activity measurement data from the wrist device through the communication circuitry, to process the received measurement data, and to cause the display screen to display the processed measurement data.

In an embodiment, the motion measurement data characterizes the physiological activity of the user.

In an embodiment, the motion measurement data comprises acceleration values in at least one dimension selected from the x-, y-, and z-axis presented in the acceleration sensor-fixed or in a reference coordinate system. The reference coordinate system may be fixed to a gyroscope or magnetometer coordinate systems, and the motion measurement data may be mapped to the reference coordinate system.

In an embodiment, the motion measurement data comprises pulse data determined from at least one acceleration values selected from selected from the x-, y-, and z-axis presented in the acceleration sensor-fixed or in a reference coordinate system.

In an embodiment, the motion measurement data comprises time distribution spent on at least one motion intensity zone, where the motion intensity may derived from at least one of acceleration values or motion pulse frequency. Below, the motion intensity zone maps to activity classes in some aspects.

In an embodiment, the motion measurement data characterizes the mechanical impact of the motion to the human body. The mechanical impact may also be referred to as mechanical training load, which characterizes the mechanical muscular load of an exercise.

In an embodiment, the heart activity measurement data measured with the heart activity transmitter 10 characterizes cardiovascular activity of the user.

In an embodiment, the heart activity measurement data comprises heart beat intervals of the heart beats, e.g. R-R intervals.

In an embodiment, the heart activity measurement data comprises heart rate values, which may or not may be averaged over a time period.

In an embodiment, the heart activity measurement data characterizes the user's energy expenditure rate or energy expenditure derived from heart rate or heart beat intervals.

In an embodiment, the heart activity measurement data characterizes time distribution spent on at least one heart rate zone having a lower limit and an upper limit.

In an embodiment, the heart activity measurement data characterizes the training load of the exercise or motion to the user's body. The training load may comprise a loading component which characterizes the physiological load of the motion and a recovery component which characterizes the user's body's trend to recover towards a normal physiological state after the motion or an exercise.

Additionally, the more versatile input interface of the portable electronic device 14 may be used by the user to control the activity monitoring relating to the motion measurement data and the heart activity measurement data, and the portable electronic device 14 may transmit commands and configuration parameters to the wrist device 12 over the wireless communication connection.

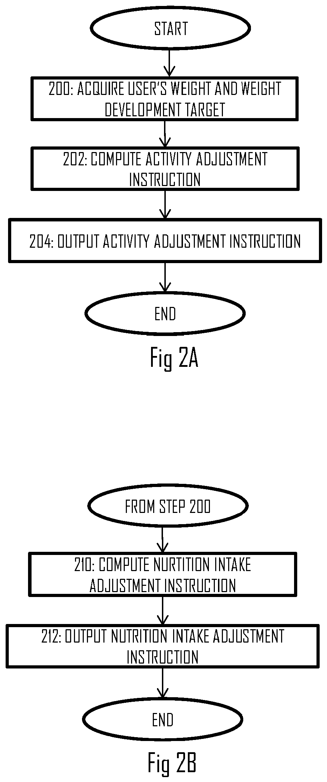

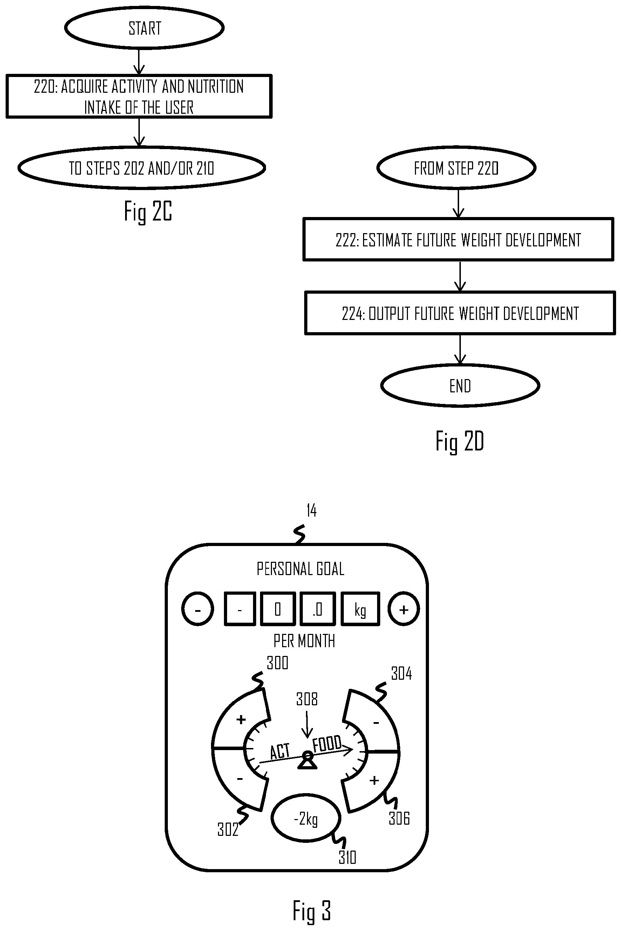

Figures 2A to 2D illustrate some embodiments of processes performed by the apparatus. Referring to Figure 2A, the process comprises acquiring (block 200) at least one weight value representing weight of the user 11 and acquiring (block 200) a weight development target for the user 11,. In block 202, the apparatus computes, on the basis of the at least one weight value and the weight development target, at least one activity adjustment instruction indicating required physical activity of the user 11. In block 204, the apparatus outputs the at least one activity adjustment instruction through an interface. Thus, the process of Figure 2 provides the apparatus with a function to compute/estimate what the activity of the user 11 should be in order to reach the weight development target value.

In an embodiment of Figure 2B, the apparatus may further compute (block 210), on the basis of the acquired at least one weight value and the acquired weight development target, at least one nutrition intake adjustment instruction indicating required nutrition intake of the user 11. This may provide a change for the user 11 to know how he/she should eat in order to reach the set weight development target. In block 212, the nutrition intake adjustment instruction may be output through the interface.

In an embodiment, as shown in block 220 of Figure 2C, the apparatus may acquire activity measurement data representing physical activity measured from the user 11 within an observation interval, and nutrition intake information of the user 11 within the observation interval. By knowing these measures, the apparatus may then proceed with more knowledge to steps 202 and/or 210 to compute the at least one activity adjustment instruction and/or the at least one nutrition intake adjustment instruction. In these cases, the at least one activity adjustment instruction may indicate how to change the physical activity of the user 11 with respect to the measured physical activity, and the at least one nutrition intake adjustment instruction may indicate how to change the nutrition intake with respect to the nutrition intake information of the user 11.

The system of Figure 1 or a system comprising some of the devices 12 to 18 of Figure 1 may be used to monitor the user's 11 daily activity and nutrition intake. The daily activity may be monitored by the activity monitoring apparatus 12 configured to measure the user's 11 physical activity. The nutrition intake may be input by the user 11. The user may input the nutrition intake through a user interface of the portable electronic device 14 or through a user interface of another apparatus, e.g. a personal computer, connected to the server 16. The nutrition intake may be input in the form which enables conversion of the nutrition intake into energy intake values.

In one embodiment of Figure 2D, the apparatus may, in block 222, estimate, on the basis of the at least one weight value, energy expenditure computed from the activity measurement data, and energy intake computed from the nutrition intake information, future weight development of the user 11. In block 223, the apparatus may output data representing the future weight development through the interface. The process of Figure 2D provides the apparatus with a function to estimate the user's 11 weight development on the basis of activity data measured from the user 11 by employing at least one sensor device. The apparatus may use the nutrition intake as another input to the weight development estimation algorithm.

In an embodiment, the apparatus may compute the at least one activity adjustment instruction and the at least one nutrition intake adjustment instruction further based on the estimated future weight development, For example, in case the future weight development, determined based on weight value, energy expenditure data, and energy intake, shows increasing weight, the guidance measures (activity and/or nutrition intake) may need to be adjusted so that the target weight development value is reached.

In an embodiment, the activity measurement data represents round-the-clock measured heart activity. In an embodiment, the activity measurement data represents activity measured with an inertial sensor round-the-clock. This may provide accuracy and reliability to the energy expenditure of the user 11.

In an embodiment, the apparatus carrying out the processes of Figure 2A-2D or any one of its embodiments described below is the server computer 16. In this embodiment, the interface may be a communication interface of the server computer 16, e.g. a network adapter providing the server computer with a communication connection to one or more computer networks. In one embodiment, the interface where the data is output is an interface of another apparatus/device, such as the interface of the portable electronic device 14. The server 16 may command the device 14 to output the data via the interface of the device 14. In another embodiment, the apparatus carrying out the process of Figure 2 or any one of its embodiments described below is the portable electronic device 14. In this embodiment, the interface may be a user interface of the device 14, e.g. a display screen or a display interface.

Figure 3 provides an illustration of the data output through the display interface of the portable electronic device 14. Referring to Figure 3, the portable electronic device 14 may be configured to execute a computer program application configured to display the effect of the measured physical activity and the nutrition intake on the user's weight. This information may be illustrated in the form of an indicator indicating the weight development, e.g. whether the weight is estimated to increase, decrease, or maintain unchanged as a result of the activity measurements and the nutrition intake. In the embodiment of Figure 3, one such indicator is provided in the form of a steelyard 308 pointing to a scale formed by elements 300, 302, 304, 406 disposed around the steelyard. The steelyard is formed by a fulcrum and a line or a plane disposed on the fulcrum. The line represents the indicator that points to the scale. The elements 300 to 306 may be provided on opposite sides of the indicator such that on each side there is provided an element 302, 304 associated with weight reduction and an element 300, 306 associated with weight increase. The elements may be provided in an opposing order on the opposite sides such that the ends of the indicator at any given time indicate elements associated with either the weight increase or decrease.

In an embodiment, Figure 3 illustrates another indicator representing the user's 11 weight development. This indicator may comprise a numeric value 310 illustrating the weight development in weight units (e.g. kilograms or pounds) within a determined time interval (e.g. one week or one month).

Let us now describe a use scenario. The user 11 may measure his/her weight by using the weighing device 18 provided with network communication capability or another weighing device, e.g. a conventional mechanical scale. The user may input the measured weight to his user account stored in the server 16 manually, or the weighing device may automatically send the measured weight to the user account. In another embodiment, the user or the weighing device inputs the measured weight to the portable electronic device 14 or to the activity monitoring apparatus 12, and the portable electronic device 14 or to the activity monitoring apparatus 12 is configured to forward the weight to the user account in the server computer 16. The server computer 16 may store identifiers, e.g. a network address or a radio link address of the devices 12, 14 associated with the user 11, and thus be able to identify the user's user account on the basis of the identifier received from the device 12, 14 transmitting the weight and/or other user-related data. When the user launches the computer program application in the portable electronic device 14 for the first time, the application may be configured to connect to the server 16 through its communication circuitry and to download user-related information from the server 16. The user-related information may comprise one or more of the following: one or more weight values, activity history, and nutrition intake history. When the user launches the application for the first time, it is possible that the server 16 stores only one weight value which is downloaded to the application and no activity or nutrition intake history is available. The portable electronic device 14 may then be configured to control the display interface to show a short animation showing the steelyard 308 or the indicator fluctuating, e.g. the steelyard tilting up and down. The indicator(s) 308, 310 may then stabilize to show no change in the weight.

When the activity measurement data and/or nutrition intake history data is available for a duration of a non-zero time interval, the application may be configured to show the estimated weight development on the basis of the energy expenditure associated with the activity measurement data and energy intake associated with the nutrition intake. As described above, the weight development may be computed in the server 16 or in the portable electronic device 14, and the indicator(s) 308, 310 may show the estimated weight development. For example, when the user launches the application, the portable electronic device 14 may be configured to control the display interface to show a short animation showing the steelyard 308 or the indicator fluctuating, e.g. the steelyard tilting up and down. The indicator(s) 308, 310 may then stabilize to show the estimated weight development, e.g. as shown in Figure 3.

With respect to the weight development, the apparatus may estimate the future weight development on the basis of the measured activity history data and the input nutrition intake history data. The algorithm executed by the apparatus may be configured to determine an observation interval, extract the measured activity history data and the input nutrition intake history data mapped to the observation interval, compute an aggregate value from the energy intake and the energy expenditure in order to determine whether or not the weight is increasing or decreasing and the amount of increaser/decrease during the observation interval. The apparatus may determine that the weight is increasing when the energy intake is higher than the energy expenditure and that the weight is decreasing when the energy intake is lower than the energy expenditure. The apparatus may determine the amount of weight development during the observation interval on the basis of the difference between the energy expenditure and the energy intake during the observation interval. The apparatus may then estimate the future weight development by determining a time period over which the future weight development is to be determined, e.g. a day, a week or a month. The apparatus may scale the weight development during the observation interval to the weight development during the time period on the basis of the ratio between the length of the observation interval and the time period. For example, if the observation interval is one week and the time period is one month, i.e. about four weeks, the apparatus may quadruple the weight development during the observation interval and output the resulting weight development value.

An embodiment provides the apparatus with guidance features. The apparatus may be configured to compute activity guidance parameters and/or nutrition intake guidance parameters. Figures 2A-2C illustrate processes according to this embodiment. The process for computing the guidance parameters comprises setting a weight development target for the user in block 200. The weight development target may specify a target for weight change over a determined time period, e.g. in kilograms or pounds per one month, a week, or two weeks. Then, in block 202, the apparatus may compute the at least one activity adjustment instruction (e.g. indicating how to change the physical activity with respect to the measured physical activity). Additionally, the apparatus may, in an embodiment, compute in block 210 the at least one nutrition intake adjustment instruction (e.g. indicating how to change the nutrition intake with respect to the nutrition intake information of the user 11). Then, the apparatus may output the activity adjustment instruction (and the nutrition intake adjustment instruction) through the interface in block(s) 204 and 212. In case the current activity of the user (within the observation period) and the current intake of the nutrition (within the observation period) is known, these may be taken into account, as illustrated in Figure 2C.

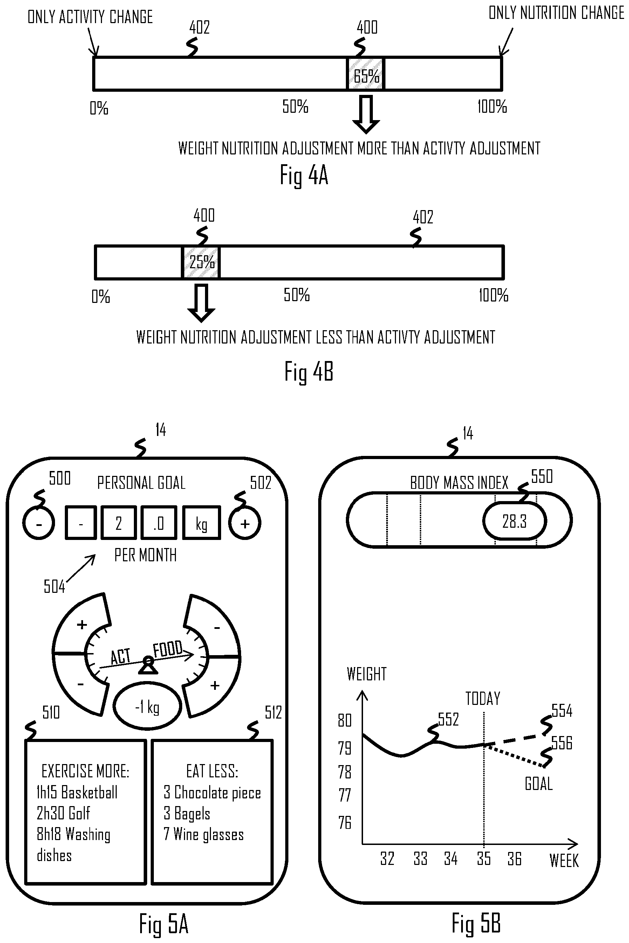

In an embodiment, as shown in Figure 4A and 4B, the user 11 may input, to the apparatus, a value defining a ratio according to which the activity adjustment and the nutrition intake adjustment are proposed for reaching the weight development target for the user 11. For example, the user 11 may give this value through a user interface of the apparatus (e.g. the server 16 or the device 14). In one example, the user may set a sliding pointer 400 on a weighting scale 402 comprising values from 0 to 100 percent. The weighting scale 402 may be a lateral or vertical sweeping touch input. Although the weighting scale 402 is depicted so that a value 100 percent means the only nutrition intake may be adjusted and not the activity, the definitions may be vice versed. As a default value, the pointer 400 may be set to 50 percent, defining that the activity adjustment and nutrition intake adjustment are given equal weight in reaching the user-set target weight value. However, in Figure 4A, the user 11 has slid the pointer 400 towards the 100 percent -end meaning (e.g. the user-set value may be 65 percent) that the nutrition intake adjustment is given more weight than the activity adjustment. In practice this may mean that the user 11 need not increase the amount of activity much but decrease the nutrition intake more. In Figure 4B, it is showed that the user 11 has slid the pointer 400 towards the other end of the weighting scale 402 (e.g. the user-set value in this case may be 65 percent), which may mean that the user 11 rather wants to increase the amount activity but decrease the nutrition intake only a little. Therefore, the embodiment of Figures 4A and 4B may provide the user 11 a change to affect the way the guidance in reaching the set weight development target is given.

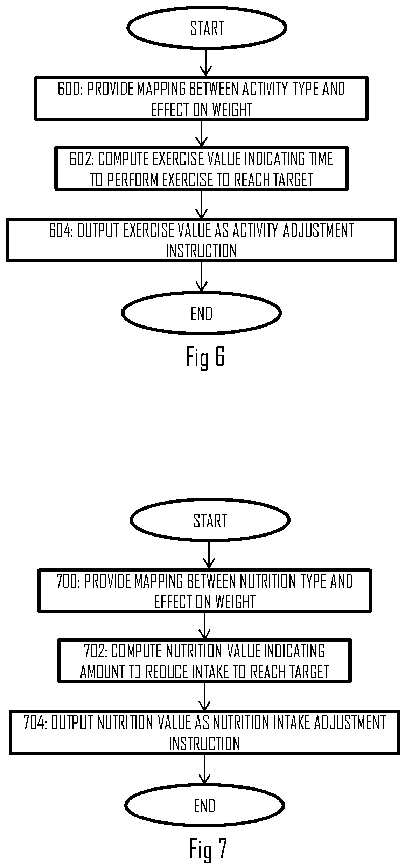

Figures 5A and 5B illustrate the display screen of the portable electronic device 14 when displaying the weight development target and the adjustment instructions. The display screen may be a touch-sensitive display screen configured to detect and decode haptic user inputs. Referring to the display view of Figure 5A, the portable electronic device 14 may be configured to display the weight development target 504 as a numeric value. The time period for the weight change may also be displayed. The display view may comprise a section 510 displaying one or more activity adjustment instructions and a section 512 displaying one or more nutrition intake adjustment instructions. The section 510 displaying one or more activity adjustment instructions may display one or more activity types and a duration value for each activity type. The duration value may indicate for how long the user 11 is instructed to perform the corresponding activity type to reach the displayed weight development target 504. The section 512 displaying one or more nutrition intake adjustment instructions may display one or more nutrition types and an amount value for each nutrition type. The amount value may indicate for how much less the user 11 is instructed to consume the corresponding nutrition type to reach the displayed weight development target 504. The amount value may be associated with the weight of the corresponding nutrition type or a number of portions of the corresponding nutrition type. Figures 6 and 7 described below provide some embodiments for computing the values in sections 510, 512.

The touch-sensitive display may comprise user interface components 500, 502 enabling the user 11 to adjust the weight development target. Upon detecting the user providing a pointing input to one of the components 500, 502, the apparatus may be configured to determine whether the input indicates increase or decrease of the weight development target 504 and, as a result of the determination, the apparatus causes the display screen to display a new weight development target corresponding to the user input. When the weight development target changes, the apparatus may be configured to reiterate the process of Figure 4 and compute new activity adjustment instructions and/or new nutrition intake instructions and display the new instructions in the sections 510, 512.

Figure 5B illustrates another display view of the portable electronic device 14. The portable electronic device 14 may provide a mechanism to switch between the display views of Figures 5A and 5B, e.g. through a haptic touch input such as a lateral or vertical sweeping touch input. The display view of Figure 5B comprises a body mass index indicator 550 indicating the user's 11 body mass index. The body mass index may be computed from the user's personal attributes input to the user account stored in the server 16 and/or to the portable electronic device 14 and from the latest weight of the user 11. Such personal attributes may comprise the user's 11 gender and height. The body mass indicator may be displayed on a scale divided into zones, wherein each zone is associated with a classification indicating normal weight, overweight, or underweight. The mass index indicator may be located in an appropriate zone according to the computed user's 11 body mass index.

The display view of Figure 5B may further illustrate the weight history in the form of a graph 552, for example. The weight history may be based on the weight measurement values input to the user account. Additionally, the weight history may be based on the activity measured with the activity monitoring apparatus 12 and/or the nutrition intake input to the user account. For example, the measured activity and the nutrition intake may be used to interpolate and/or extrapolate weight values between the measured weight values. The measured weight values may thus serve as anchor points for the graph 552. The graph 552 may show the past weight history. The weight history until the current time instant ("Today") may be used to estimate future weight development with the current general activity level and nutrition intake level. The apparatus may compute the future weight development in the above-described manner and display a graph 554 showing the estimated weight development. Additionally, the apparatus may compute and display another graph 556 showing future weight development if the user reaches the weight development target 504. This graph 556 may be displayed as a line between the current weight and a weight value matching with the weight development target.

Figure 6 illustrates an embodiment of a function executed in block 202, wherein the at least one activity adjustment instruction is computed. Referring to Figure 6, the process in block 402 may comprise providing a mapping between each of a plurality of activity types to a value describing how exercising the activity type affects the weight (block 600). The mapping may be provided in a database stored in a memory device of the apparatus or in a memory device accessible to the apparatus. The mapping may indicate, for each activity type, energy consumption per time unit associated with said activity type. The apparatus may compute, on the basis of the mapping and the weight development target for an activity type, an exercise value describing a time period indicating how much more the user needs to perform said activity type in order to reach the weight development target (block 602). The computation in block 602 may comprise determining a difference between the current weight and a target weight associated with the weight development target, computing energy expenditure needed to achieve the difference, and determining for each activity type, a time period needed exercising before the energy expenditure is reached. The time period may then be output as the exercise value. The apparatus may then output, through the interface, said activity adjustment instruction comprising the exercise value and an identifier of said activity type. The activity type and the exercise value may then be displayed in the section 510 of the portable electronic device 14.

In an embodiment of block 602, the exercise value describing the time period is computed by employing as an input user's personal attributes including at least one of weight and gender. The user's weight and gender may affect the energy consumption and, therefore, more accurate estimate of the energy expenditure per time unit of the activity type may be achieved by using the personal attributes that affect the energy expenditure. The personal attributes may be acquired from the user's 11 user account or they may be input to the apparatus by the user 11.

The activity types for which the exercise value is computed may comprise at least some of the following: walking, running, cycling, swimming, doing household chores, and playing a determined game.

Figure 7 illustrates another embodiment of a function executed in block 210, wherein the at least one nutrition intake adjustment instruction is computed. Referring to Figure 7, said computing the at least one nutrition intake adjustment instruction comprises providing a mapping between each of a plurality of nutrition types to a value describing how intake of the nutrition type affects the weight (block 700). The mapping may be provided in a database stored in a memory device of the apparatus or in a memory device accessible to the apparatus. The mapping may indicate, for each nutrition type, energy intake per unit associated with said nutrition type. The apparatus may compute, on the basis of the mapping and the weight development target for a nutrition type, a nutrition value describing an amount of said nutrition type the user needs to intake less in order to reach the weight development target (block 702). The nutrition value may be computed through energy intake per unit of said nutrition type. The unit may be a weight unit or a portion unit. In block 704, the apparatus may output, through the interface, said nutrition intake adjustment instruction comprising said nutrition value and an identifier of said nutrition type. The nutrition type(s) and associated nutrition value(s) may then be displayed in the section 512 of the portable electronic device 14.

In an embodiment of block 702, the nutrition value describing the amount of nutrition type is computed by employing as an input user's personal attributes including at least one of weight and gender. The personal attributes may be acquired from the user's 11 user account or they may be input to the apparatus by the user 11.

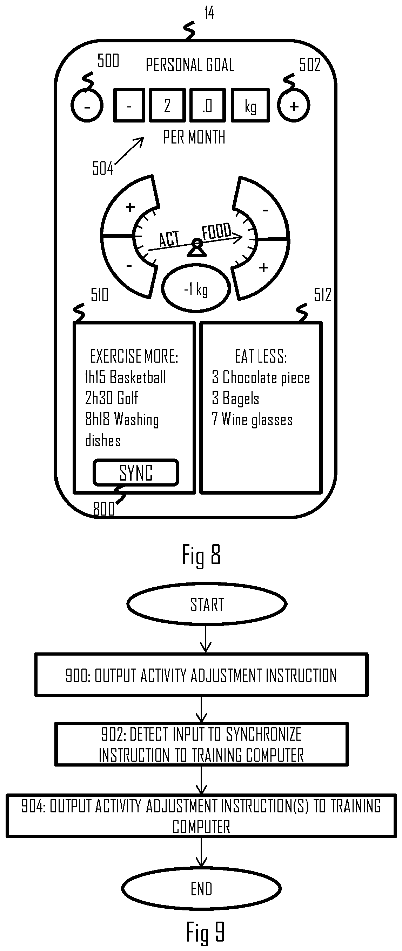

Figures 8 and 9 illustrate an embodiment where said activity adjustment instruction output through the interface reconfigures at least one activity target of a personal training computer carried by the user. The personal training computer may be the activity monitoring device 12 or the portable electronic device 14. Referring to Figures 8, the display screen of the portable electronic device may comprise a component 800 selectable by the user 11 through a user input, e.g. a haptic input. Referring to Figure 9, the portable electronic device 14 may output the activity adjustment instructions in the section 510, as described above. When the portable electronic device 14 detects the user input selecting the component 800 (block 902), the portable electronic device may reconfigure one or more activity targets for the user (block 904). The reconfiguration may comprise implementing the activity adjustment instructions to the activity targets, e.g. synchronizing the activity adjustment instructions to the activity targets. The activity targets may define targets for accumulation of activity for one or more activity types during a determined time interval, e.g. a target for walking may be two hours per day. The reconfiguration of the target(s) may comprise modifying the old target(s) with the exercise value. For example, if an old target is two hours of walking per day and the exercise value indicates that 30 minutes more walking per day is needed to reach the weight development target, the portable electronic device may set a new target of two hours and 30 minutes of walking per day. On the other hand, if an old target is five hours of high activity time (measured activity above a determined threshold) per day and the exercise value indicates that one hour less activity is needed to reach the weight development target, the portable electronic device 14 may set a new target of four hours of high activity time per day.

In an embodiment where the activity targets are used and displayed to the user in the activity monitoring device 12, block 904 may comprise outputting an instruction to update the activity targets with the activity adjustment instructions, wherein the instruction may be communicated from the portable electronic device 14 to the activity monitoring device 12 over a wired or wireless communication connection.

In an embodiment where the activity targets are used and displayed to the user in the portable electronic device 14, block 904 may comprise outputting the activity adjustment instructions to a computer program module executed in the portable electronic device, managing the activity targets, and monitoring activity accumulation performed by the user 11.

In an embodiment, the apparatus (the server 16 or portable electronic device 14) may provide, through the interface, an instruction for the user 11 to measure his/her weight upon detecting a predetermined event. This may be beneficial in that the user 11 remember to measure this/her weight. Knowing the current weight reliably and up-to-date, provides for more accurate instructions in steps 202 (and 210) in reaching the set weight development target.

In an embodiment, the predetermined event may be defined as a time of a clock. For example, the apparatus may give a command "go to scale" every day at 10 o'clock. In one embodiment, the apparatus may detect the person waking up, and give the instructions right after waking up.

In an embodiment, the predetermined event may be defined in measured activity level. For example, the apparatus may give a command "go to scale" every time the measured activity level increase over an activity threshold. The activity level may be based on the activity measurement data.

In an embodiment, the instruction is given as a written text indication on the interface. In an embodiment, the instruction is given as a vibration alert. In an embodiment, the instruction is given as an audio alert. The instruction may be given in the portable electronic device 14 or in the activity monitoring device 12.

In one embodiment, there are intermediate targets in the weight development. The user 11 may him/herself set the weight development intermediate targets or they may be automatically set on the basis of the difference between the current weight and the target weight. Once the user 11 meets an intermediate target, the user 11 may receive a notification of this. The notification may come through the interface. This may increase the motivation of the user 11 to reach the final weight development target.

In one embodiment, at least one statistical parameter is determined from data stored in the server 16. The server 16 may store data for a plurality of users, the data representing the weight or weight development of the plurality of users. The at least one statistical parameter may be an average weight of certain group of users, an average weight development of a certain group of users, and/or an average amount of weight lost. The certain group of users (i.e. a subgroup of users) may be a group of users having a predetermined age range, such as less than 20 years, between 21-30 years, between 31-40 years, between 41-50 years, between 51-60 years, or over 60 years, to mention only a few non-limiting age range examples. The certain group of users may also be selected according to other criteria, such as according to gender, according to initial weight value, according to the area (country, city, village) in which the persons live, according to how much exercise has been performed, according to ratio according to which the activity adjustment and the nutrition intake adjustment are proposed for reaching the weight development target, to mention only a few examples. In an embodiment, the certain group may also be self-established so that only friends of a given user are members of the group. In an embodiment, only users who have set a target to lose weight are members of the certain group.

The determined at least one statistical parameter may then be compared against the corresponding parameter of the user in question. For example, let us assume that the weight loss of the user is 10 kg, while the average weight loss of the group is only 5kg. Then an indication of the difference may be provided to the user. Further, a credit may be provided to the user for the work well done. There may be thresholds for giving the credits, such as a credit is given only when the user has lost at least as much weight as the average weight loss is. The credit may be a monetary compensation, a training product gift, for example. The credits may be accumulated and when a certain amount of credits are obtained, the user may change the credits to a certain product, gift, monetary compensation, etc.

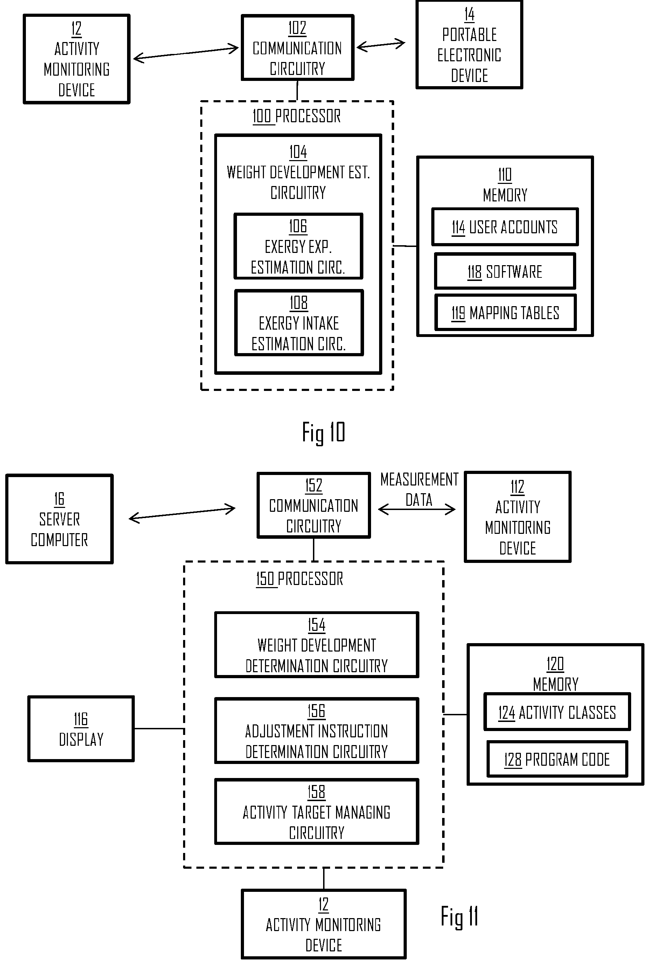

Figure 10 illustrates a block diagram of a structure of an apparatus according to an embodiment of the invention. The apparatus may be applicable to or comprised in the server computer 16. The apparatus may comprise at least one processor 100 or processing circuitry and at least one memory 110 including a computer program code 118, wherein the at least one memory and the computer program code are configured, with the at least one processor, to cause the apparatus to carry out the functions described above in connection with the server computer, e.g. any one of the processes of Figures 2, 4, 6, and 7. The apparatus may comprise a communication circuitry 102 configured to handle the connections with the portable electronic device 14, activity monitoring device 12 and, in some embodiments, the weighing device 18. The communication circuitry 102 may be configured to establish, maintain, and terminate connections with the devices and transfer data over the connections. For example, the activity measurement data may be transferred through the communication circuitry 102 and stored in a user account 114 comprised in the memory 110. The activity measurement data may comprise at least one of the following: the heart activity measurement data, the motion measurement data, and one or more activity metric(s) computed by the activity monitoring device 12 on the basis of the heart activity measurement data and/or the motion measurement data. In an embodiment, the server computer 16 receives raw measurement data as the activity data from the activity monitoring device 12 and computes the activity metric(s) from the raw measurement data. In other embodiments, the portable electronic device 12 receives the activity metric(s) as computed by the wrist device. The one or more activity metrics may comprise a heart rate, energy consumption, heart rate accumulation over time, or an activity class. An activity class may represent quantization of the motion activity metric and/or heart activity metric. The range of motion activity metrics and/or heart activity metrics may be divided into a plurality of zones, and each zone may represent an activity class. Lowest motion activity metrics and/or heart activity metrics may be allocated to the lowest activity class and vice versa. The portable electronic device 14 and/or the server computer 16 may compute further activity metric(s) from the received activity metric(s), e.g. energy expenditure from the activity accumulation.

The processor 100 may comprise a weight development estimation circuitry 104 configured to estimate user's 11 weight development in the above-described manner. The weight development estimation circuitry 104 may comprise an energy expenditure estimation circuitry 106 configured to receive as an input the activity measurement data received through the communication circuitry 102 from the activity monitoring device. In some embodiments, the activity measurement data may be routed through the portable electronic device 14 and/or another mediating device. The activity measurement data may be stored in the user account 114 in the above-described manner. As described above, the energy expenditure estimation circuitry 106 may be configured to determine the user's personal attributes and use them as another input. The energy expenditure estimation circuitry 106 may compute the effect of the measured activity on the user's weight loss by employing an appropriate mapping between the energy expenditure computed from the activity measurement data and the weight loss. The mapping may be stored in a mapping table 119 in the memory 110. In embodiments where the energy expenditure estimation circuitry 106 carries out a part of block 202 and/or the process of Figure 6, the energy expenditure estimation circuitry 106 may be configured to use the weight development target as a further input and compute, on the basis of said mapping between the energy expenditure computed from the activity measurement data and the weight loss, the amount of energy the user has to expend in order to change weight from the latest value of the weight to the weight development target. The mapping tables 119 may further store information on energy expenditure per time unit for a plurality of activity types. The energy expenditure estimation circuitry 106 may use this information to determine, for one or more activity types, duration for how long the user needs to perform said one or more activity types in order to cause the weight change. The energy expenditure estimation circuitry 106 may then store the duration as the activity adjustment instruction in the user account 114 or output the instruction to the communication circuitry 102.

The weight development estimation circuitry 104 may comprise a nutrition intake estimation circuitry 108 configured to receive as an input the nutrition intake data received through the communication circuitry 102 or through a user interface from the user 11. The nutrition intake data may be stored in the user account 114 in the above-described manner. The nutrition intake estimation circuitry 108 may compute the effect of the nutrition intake on the user's weight loss by employing an appropriate mapping between the energy intake computed from the nutrition intake data and the weight increase. The mapping may be stored in the mapping table 119. In embodiments where the nutrition intake estimation circuitry 108 carries out a part of block 210 and/or the process of Figure 7, the nutrition intake estimation circuitry 108 may be configured to use the weight development target as a further input and compute, on the basis of said mapping between the energy intake computed from the nutrition intake data and the weight increase, the change in the amount of energy the user has to intake in order to change weight from the latest value of the weight to the weight development target. The mapping tables 119 may further store information on energy intake per nutrition unit for a plurality of nutrition types. The nutrition intake estimation circuitry 108 may use this information to determine, for one or more nutrition types, a value indicating a change in an amount the user needs to intake said one or more nutrition types in order to cause the weight change. The nutrition intake estimation circuitry 108 may then store the value as the nutrition intake adjustment instruction in the user account 114 or output the instruction to the communication circuitry 102.

Whenever, the portable electronic device 14 or the activity monitoring device 12 requests for a specific information element from the user account 114 or an update of the user account, the processor 100 may be configured to update the user account with further data received from the device(s) 12, 14 and/or to send data contents of the user account 114 to the device(s) 12, 14, depending on the type of the request. In an embodiment where the weight development estimation circuitry 104 is provided in the device(s) 12, 14, the processor may simply manage and transfer contents of the user accounts 114.

Figure 11 illustrates a block diagram of a structure of an apparatus according to an embodiment of the invention. The apparatus may be applicable to or comprised in the portable electronic device 14, or the apparatus may be the portable electronic device 14. The apparatus may comprise at least one processor 150 or processing circuitry and at least one memory 120 including a computer program code 128, wherein the at least one memory and the computer program code are configured, with the at least one processor, to cause the apparatus to carry out the functions described above in connection with the portable electronic device 14, e.g. any one of the processes of Figures 2, 4, 6, and 7. The apparatus may comprise a communication circuitry 152 configured to handle the connections with the server computer 16, activity monitoring device 12 and, in some embodiments, the weighing device 18. At least some of the connection s may comprise a wireless communication link, e.g. a short range communication link and/or a long range communication link. An example of the short range communication link is a Bluetooth® radio link, while an example of the long range communication link is a cellular radio link such as Long-Term Evolution (LTE) of Universal Mobile Telecommunication System (UMTS). The communication circuitry 152 may be configured to establish, maintain, and terminate connections with the devices and transfer data over the connections.

The apparatus may further comprise a display configured, under the control of the processor 150, to provide at least some of the display views illustrated in the Figures 3, 5A, 5B, and 8.

In some embodiments, the apparatus may comprise the activity monitoring device 12. For example, the apparatus may comprise at least one motion sensor or another sensor configured to measure the activity measurement data. The apparatus may further be connected, through the communication circuitry 152, to another activity monitoring device 112, e.g. the wrist device or a heart activity transmitter.

The processor 150 may be configured to execute the above-described computer program application configured to display the effect of the measured physical activity and the nutrition intake on the user's weight. The execution of the application may trigger a weight development determination circuitry 154 to acquire, from the server computer 16 and/or from the memory 120, the weight development history of the user 11 and a future weight development estimate. In an embodiment where the weight development estimation circuitry 104 of the server computer 16 computes this data, the weight development determination circuitry 154 may download the data from the server computer and configure the display 116 to display the data in the form illustrated in display views 3, 5A, 5B, 8. In another embodiment, the weight development determination circuitry 154 may comprise the weight development estimation circuitry 104.

The processor 150 may further comprise an adjustment instruction determination circuitry 156 configured to acquire the above-described activity adjustment instructions and/or the nutrition intake adjustment instructions and configured the display 116 to display one or both of the sections 510, 512. Depending on the embodiment, the adjustment instruction determination circuitry 156 may acquire the adjustment instructions from the server computer or from the weight development determination circuitry 154.

The adjustment instructions may indicate only the change to the current behaviour of the user. The processor 150 may comprise an activity target managing circuitry 158 configured to manage activity targets and to implement the instructed changes comprised in the activity adjustment instructions to real activity targets, wherein the activity targets represent the total energy expenditure required of the user 11 within a time period. Upon receiving an instruction to change the activity targets through a user interface, for example, the activity target managing circuitry 158 may add one or more values comprised in the activity adjustment instructions and associated with one or more activity types to current activity targets of the corresponding activity type. The activity target managing circuitry 158 may then cause the display 116 to display the updated activity targets.

As used in this application, the term 'circuitry' refers to all of the following: (a) hardware-only circuit implementations such as implementations in only analog and/or digital circuitry; (b) combinations of circuits and software and/or firmware, such as (as applicable): (i) a combination of processor(s) or processor cores; or (ii) portions of processor(s)/software including digital signal processor(s), software, and at least one memory that work together to cause an apparatus to perform specific functions; and (c) circuits, such as a microprocessor(s) or a portion of a microprocessor(s), that require software or firmware for operation, even if the software or firmware is not physically present.

This definition of 'circuitry' applies to all uses of this term in this application. As a further example, as used in this application, the term "circuitry" would also cover an implementation of merely a processor (or multiple processors) or portion of a processor, e.g. one core of a multi-core processor, and its (or their) accompanying software and/or firmware. The term "circuitry" would also cover, for example and if applicable to the particular element, a baseband integrated circuit, an application-specific integrated circuit (ASIC), and/or a field-programmable grid array (FPGA) circuit for the apparatus according to an embodiment of the invention.

The processes or methods described in Figures 2 to 9 may also be carried out in the form of a computer process defined by a computer program. The computer program may be in source code form, object code form, or in some intermediate form, and it may be stored in some sort of carrier, which may be any entity or device capable of carrying the program. Such carriers include transitory and/or non-transitory computer media, e.g. a record medium, computer memory, read-only memory, electrical carrier signal, telecommunications signal, and software distribution package. Depending on the processing power needed, the computer program may be executed in a single electronic digital processing unit or it may be distributed amongst a number of processing units.

The present invention is applicable to activity monitoring systems. The algorithms used may develop over time, and such development may require extra changes to the described embodiments.

## Claims

1. A method comprising:
acquiring, in an apparatus, at least one weight value representing weight of a user;
acquiring, in an apparatus, a weight development target for the user;
acquiring activity measurement data representing physical activity measured from the user within an observation interval;
acquiring nutrition intake information of the user within the observation interval;
computing, on the basis of the at least one weight value and the weight development target, at least one activity adjustment instruction indicating required physical activity of the user; and
outputting the activity adjustment instruction through an interface
computing, on the basis of the at least one weight value and the weight development target, at least one nutrition intake adjustment instruction indicating required nutrition intake of the user; and
outputting the nutrition intake adjustment instruction through the interface,
**characterized by** the method further comprising as performed in the apparatus:
estimating, on the basis of the at least one weight value, energy expenditure computed from the activity measurement data, and energy intake computed from the nutrition intake information, future weight development of the user;
outputting data representing the future weight development through the interface; and
computing the at least one activity adjustment instruction and the at least one nutrition intake adjustment instruction further based on the estimated future weight development, wherein the at least one activity adjustment instruction indicates how to change the physical activity with respect to the measured physical activity and the at least one nutrition intake adjustment instruction indicates how to change the nutrition intake with respect to the nutrition intake information of the user.

2. The method of claim 1, further comprising:
receiving, as an input, a value defining a ratio according to which the activity adjustment and the nutrition intake adjustment are proposed for reaching the weight development target for the user.

3. The method of claim 1 or 2, wherein the activity measurement data represents round-the-clock measured heart activity and/or activity measured with an inertial sensor round-the-clock.

4. The method of any of claims 1 to 3, wherein said computing the at least one activity adjustment instruction comprises:
providing a mapping between each of a plurality of activity types to a value describing how exercising the activity type affects the weight;
computing, on the basis of the mapping and the weight development target for an activity type, an exercise value describing a time period the user needs to perform said activity type in order to reach the weight development target; and
outputting, through the interface, said activity adjustment instruction comprising the exercise value and an identifier of said activity type.

5. The method of claim 4, wherein the exercise value describing the time period is computed by employing as an input user's personal attributes including at least one of weight, gender, activity level, maximum performance level.

6. The method of any of claims 1 to 5, wherein said computing the at least one nutrition intake adjustment instruction comprises:
providing a mapping between each of a plurality of nutrition types to a value describing how intake of the nutrition type affects the weight;
computing, on the basis of the mapping and the weight development target for a nutrition type, a nutrition value describing an amount of said nutrition type the user needs to intake less in order to reach the weight development target; and
outputting, through the interface, said nutrition intake adjustment instruction comprising said nutrition value and an identifier of said nutrition type.

7. The method of claim 6, wherein the nutrition value is computed by employing as an input user's personal attributes including at least one of weight, gender, activity level, maximum performance level.

8. The method of any of claims 1 to 7, wherein said activity adjustment instruction output through the interface reconfigures at least one activity target of a personal training computer carried by the user.

9. The method of any of claims 1 to 8, further comprising:
providing, through the interface and upon detecting a predetermined event,, an instruction for the user to measure his/her weight.

10. An apparatus comprising:
means for acquiring at least one weight value representing weight of a user;
means for acquiring a weight development target for the user;
means for acquiring activity measurement data representing physical activity measured from the user within an observation interval;
means for acquiring nutrition intake information of the user within the observation interval;
means for computing, on the basis of the at least one weight value and the weight development target, at least one activity adjustment instruction indicating required physical activity of the user; and
means for outputting the activity adjustment instruction through an interface of the apparatus
means for computing, on the basis of the at least one weight value and the weight development target, at least one nutrition intake adjustment instruction indicating required nutrition intake of the user; and
means for outputting the nutrition intake adjustment instruction through the interface,
**characterized by** the apparatus further comprising:
means for estimating, on the basis of the at least one weight value, energy expenditure computed from the activity measurement data, and energy intake computed from the nutrition intake information, future weight development of the user; and
means for outputting data representing the future weight development through the interface; and
means for computing the at least one activity adjustment instruction and the at least one nutrition intake adjustment instruction further based on the estimated future weight development, wherein the at least one activity adjustment instruction indicates how to change the physical activity with respect to the measured physical activity and the at least one nutrition intake adjustment instruction indicates how to change the nutrition intake with respect to the nutrition intake information of the user.

11. The apparatus of claim 10, further comprising means for carrying out all the steps of the method according to any preceding claim 2 to 9.

12. A computer program product embodied on a distribution medium readable by a computer and comprising program instructions which, when running on a computer, execute the method according to any preceding claim 1 to 9.

## Patentansprüche

1. Verfahren, das Folgendes umfasst:
Erfassen von mindestens einem Gewichtswert, der ein Gewicht eines Benutzers repräsentiert, in einer Vorrichtung;
Erfassen eines Gewichtsentwicklungsziels für den Benutzer in einer Vorrichtung;
Erfassen von Aktivitätsmessdaten, die eine physische Aktivität repräsentieren, die in einem Beobachtungsintervall am Benutzer gemessen wird;
Erfassen von Nahrungsaufnahmeinformationen des Benutzers im Beobachtungsintervall;
Berechnen von mindestens einer Aktivitätsanpassungsanweisung, die eine erforderliche physische Aktivität des Benutzers anzeigt, auf Basis des mindestens einen Gewichtswerts und des Gewichtsentwicklungsziels; und
Ausgeben der Aktivitätsanpassungsanweisung über eine Schnittstelle
Berechnen von mindestens einer Nahrungsaufnahmeanpassungsanweisung, die eine erforderliche Nahrungsaufnahme des Benutzers anzeigt, auf Basis des mindestens einen Gewichtswerts und des Gewichtsentwicklungsziels; und
Ausgeben der Nahrungsaufnahmeanpassungsanweisung über die Schnittstelle,
**dadurch gekennzeichnet, dass** das Verfahren das Durchführen von Folgendem in der Vorrichtung umfasst:
Schätzen einer künftigen Gewichtsentwicklung des Benutzers auf Basis des mindestens einen Gewichtswerts, eines Energieverbrauchs, der aus den Aktivitätsmessdaten berechnet wird, und einer Energieaufnahme, die aus den Nahrungsaufnahmeinformationen berechnet wird;
Ausgeben von Daten, die die künftige Gewichtsentwicklung repräsentieren, über die Schnittstelle; und
Berechnen der mindestens einen Aktivitätsanpassungsanweisung und der mindestens einen Nahrungsaufnahmeanpassungsanweisung ferner auf Basis der geschätzten künftigen Gewichtsentwicklung, wobei die mindestens eine Aktivitätsanpassungsanweisung anzeigt, wie die physische Aktivität mit Bezug auf die gemessene physische Aktivität zu ändern ist, und die mindestens eine Nahrungsaufnahmeanpassungsanweisung anzeigt, wie die Nahrungsaufnahme mit Bezug auf die Nahrungsaufnahmeinformationen des Benutzers zu ändern ist.

2. Verfahren nach Anspruch 1, das ferner Folgendes umfasst:
Empfangen eines Wertes, der ein Verhältnis definiert, gemäß dem die Aktivitätsanpassung und die Nahrungsaufnahmeanpassung hinsichtlich des Erreichens des Gewichtsentwicklungsziels des Benutzers vorgeschlagen werden, als eine Eingabe.

3. Verfahren nach Anspruch 1 oder 2, wobei die Aktivitätsmessdaten eine rund um die Uhr gemessene Herzaktivität und/oder eine mit einem Inertialsensor rund um die Uhr gemessene Aktivität repräsentieren.

4. Verfahren nach einem der Ansprüche 1 bis 3, wobei das Berechnen der mindestens einen Aktivitätsanpassungsanweisung Folgendes umfasst:
Bereitstellen einer Zuordnung zwischen jedem einer Vielzahl von Aktivitätstypen zu einem Wert, der beschreibt, wie das Ausüben des Aktivitätstyps das Gewicht beeinträchtigt;
Berechnen eines Übungswertes, der eine Zeitperiode beschreibt, in der der Benutzer den Aktivitätstyp durchführen muss, um das Gewichtsentwicklungsziel zu erreichen, auf Basis der Zuordnung und des Gewichtsentwicklungsziels für einen Aktivitätstyp; und
Ausgeben der Aktivitätsanpassungsanweisung, die den Übungswert und eine Kennung des Aktivitätstyps umfasst, über die Schnittstelle.

5. Verfahren nach Anspruch 4, wobei der Übungswert, der die Zeitperiode beschreibt, durch Einsetzen von persönlichen Attributen eines Benutzers, die mindestens eines von Gewicht, Geschlecht, Aktivitätsniveau, maximales Leistungsniveau beinhalten, als eine Eingabe berechnet wird.

6. Verfahren nach einem der Ansprüche 1 bis 5, wobei das Berechnen der mindestens einen Nahrungsaufnahmeanpassungsanweisung Folgendes umfasst:
Bereitstellen einer Zuordnung zwischen jedem einer Vielzahl von Nahrungstypen zu einem Wert, der beschreibt, wie die Aufnahme des Nahrungstyps das Gewicht beeinträchtigt;
Berechnen eines Nahrungswertes, der eine Menge des Nahrungstyps beschreibt, die ein Benutzer weniger aufnehmen muss, um das Gewichtsentwicklungsziel zu erreichen, auf Basis der Zuordnung und des Gewichtsentwicklungsziels für einen Nahrungstyp; und
Ausgeben der Nahrungsaufnahmeanpassungsanweisung, die den Nahrungswert und eine Kennung des Nahrungstyps umfasst, über die Schnittstelle.

7. Verfahren nach Anspruch 6, wobei der Nahrungswert durch Einsetzen von persönlichen Attributen eines Benutzers, die mindestens eines von Gewicht, Geschlecht, Aktivitätsniveau, maximales Leistungsniveau beinhalten, als eine Eingabe berechnet wird.

8. Verfahren nach einem der Ansprüche 1 bis 7, wobei die Aktivitätsanpassungsanweisung, die über die Schnittstelle ausgegeben wird, mindestens ein Aktivitätsziel eines persönlichen Trainingscomputers, der vom Benutzer getragen wird, neu ausgelegt.

9. Verfahren nach einem der Ansprüche 1 bis 8, das ferner Folgendes umfasst:
Bereitstellen einer Anweisung für den Benutzer, sein Gewicht zu messen, über die Schnittstelle und nach Detektieren eines vorbestimmten Ereignisses.

10. Vorrichtung, die Folgendes umfasst:
Mittel zum Erfassen von mindestens einem Gewichtswert, der ein Gewicht eines Benutzers repräsentiert;
Mittel zum Erfassen eines Gewichtsentwicklungsziels für den Benutzer;
Mittel zum Erfassen von Aktivitätsmessdaten, die eine physische Aktivität repräsentieren, die in einem Beobachtungsintervall am Benutzer gemessen wird;
Mittel zum Erfassen von Nahrungsaufnahmeinformationen des Benutzers im Beobachtungsintervall;
Mittel zum Berechnen von mindestens einer Aktivitätsanpassungsanweisung, die eine erforderliche physische Aktivität des Benutzers anzeigt, auf Basis des mindestens einen Gewichtswerts und des Gewichtsentwicklungsziels; und
Mittel zum Ausgeben der Aktivitätsanpassungsanweisung über eine Schnittstelle der Vorrichtung,
Mittel zum Berechnen von mindestens einer Nahrungsaufnahmeanpassungsanweisung, die eine erforderliche Nahrungsaufnahme des Benutzers anzeigt, auf Basis des mindestens einen Gewichtswerts und des Gewichtsentwicklungsziels; und
Mittel zum Ausgeben der Nahrungsaufnahmeanpassungsanweisung über die Schnittstelle,
**dadurch gekennzeichnet, dass** die Vorrichtung ferner Folgendes umfasst:
Mittel zum Schätzen einer künftigen Gewichtsentwicklung des Benutzers auf Basis des mindestens einen Gewichtswerts, eines Energieverbrauchs, der aus den Aktivitätsmessdaten berechnet wird, und einer Energieaufnahme, die aus den Nahrungsaufnahmeinformationen berechnet wird; und
Mittel zum Ausgeben von Daten, die die künftige Gewichtsentwicklung repräsentieren, über die Schnittstelle; und
Mittel zum Berechnen der mindestens einen Aktivitätsanpassungsanweisung und der mindestens einen Nahrungsaufnahmeanpassungsanweisung ferner auf Basis der geschätzten künftigen Gewichtsentwicklung, wobei die mindestens eine Aktivitätsanpassungsanweisung anzeigt, wie die physische Aktivität mit Bezug auf die gemessene physische Aktivität zu ändern ist, und die mindestens eine Nahrungsaufnahmeanpassungsanweisung anzeigt, wie die Nahrungsaufnahme mit Bezug auf die Nahrungsaufnahmeinformationen des Benutzers zu ändern ist.

11. Vorrichtung nach Anspruch 10, die ferner Mittel zum Umsetzen aller Schritte des Verfahrens nach einem der vorhergehenden Ansprüche 2 bis 9 umfasst.

12. Computerprogrammprodukt, das auf einem Verteilungsmedium enthalten ist, das von einem Computer lesbar ist und Programmanweisungen umfasst, die, wenn sie auf einem Computer laufen, das Verfahren gemäß einem der vorhergehenden Ansprüche 1 bis 9 ausführen.

## Revendications

1. Procédé comprenant :
l'acquisition, sur un appareil, d'au moins une valeur de poids représentant le poids d'un utilisateur ;
l'acquisition, sur un appareil, d'un objectif d'évolution de poids pour l'utilisateur ;
l'acquisition de données de mesure d'activité représentant une activité physique mesurée chez l'utilisateur dans un intervalle d'observation ;
l'acquisition d'informations d'apport nutritionnel de l'utilisateur dans l'intervalle d'observation ;
le calcul, en se basant sur l'au moins une valeur de poids et l'objectif d'évolution de poids, d'au moins une instruction d'ajustement d'activité indiquant une activité physique requise de l'utilisateur ; et
la sortie de l'instruction d'ajustement d'activité par le biais d'une interface calculant, en se basant sur l'au moins une valeur de poids et l'objectif d'évolution de poids, au moins une instruction d'ajustement d'apport nutritionnel indiquant un apport nutritionnel requis de l'utilisateur ; et
la sortie de l'instruction d'ajustement d'apport nutritionnel par le biais de l'interface,
**caractérisé en ce que** le procédé comprend en outre, tels que réalisés sur l'appareil :
l'estimation, en se basant sur l'au moins une valeur de poids, une dépense énergétique calculée à partir des données de mesure d'activité et un apport énergétique calculé à partir des informations d'apport nutritionnel, d'une évolution future de poids de l'utilisateur ;
la sortie de données représentant l'évolution future de poids par le biais de l'interface ; et
le calcul de l'au moins une instruction d'ajustement d'activité et de l'au moins une instruction d'ajustement d'apport nutritionnel, en se basant en outre sur l'évolution future de poids estimée, dans lequel l'au moins une instruction d'ajustement d'activité indique comment modifier l'activité physique par rapport à l'activité physique mesurée, et l'au moins une instruction d'ajustement d'apport nutritionnel indique comment modifier l'apport nutritionnel par rapport aux informations d'apport nutritionnel de l'utilisateur.

2. Procédé de la revendication 1, comprenant en outre :
la réception, comme entrée, d'une valeur définissant un rapport selon lequel l'ajustement d'activité et l'ajustement de l'apport nutritionnel sont proposés pour atteindre l'objectif d'évolution de poids pour l'utilisateur.

3. Procédé de la revendication 1 ou 2, dans lequel les données de mesure d'activité représentent l'activité cardiaque mesurée 24 heures sur 24 et/ou l'activité mesurée avec un capteur inertiel 24 heures sur 24.

4. Procédé de l'une des revendications 1 à 3, dans lequel ledit calcul de l'au moins une instruction d'ajustement d'activité comprend :
la fourniture d'une mise en correspondance entre chacun d'un pluralité de types d'activité et une valeur décrivant comment l'exercice du type d'activité affecte le poids ;
le calcul, en se basant sur la mise en correspondance et l'objectif d'évolution de poids pour un type d'activité, d'une valeur d'exercice décrivant une période de temps dont l'utilisateur a besoin pour réaliser ledit type d'activité afin d'atteindre l'objectif d'évolution de poids ; et
la sortie, par le biais de l'interface, de ladite instruction d'ajustement d'activité comprenant la valeur d'exercice et un identifiant dudit type d'activité.

5. Procédé de la revendication 4, dans lequel la valeur d'exercice décrivant la période de temps est calculée en employant comme entrée, des attributs personnels de l'utilisateur comportant au moins l'un parmi le poids, le sexe, le niveau d'activité, le niveau de performance maximum.

6. Procédé de l'une des revendications 1 à 5, dans lequel ledit calcul de l'au moins une instruction d'ajustement d'apport nutritionnel comprend :
la fourniture d'une mise en correspondance entre chacun d'une pluralité de types de nutrition et une valeur décrivant comment l'apport du type de nutrition affecte le poids ;
le calcul, en se basant sur la mise en correspondance et l'objectif d'évolution de poids pour un type de nutrition, d'une valeur nutritionnelle décrivant une quantité dudit type de nutrition dont l'utilisateur a besoin pour moins d'apport afin d'atteindre l'objectif d'évolution de poids ; et
la sortie, par le biais de l'interface, de ladite instruction d'ajustement d'apport nutritionnel comprenant ladite valeur nutritionnelle et un identifiant dudit type de nutrition.

7. Procédé de la revendication 6, dans lequel la valeur nutritionnelle est calculée en employant comme entrée, des attributs personnels de l'utilisateur comportant au moins l'un parmi le poids, le sexe, le niveau d'activité, le niveau de performance maximum.

8. Procédé de l'une des revendications 1 à 7, dans lequel ladite instruction d'ajustement d'activité délivrée par le biais de l'interface reconfigure au moins un objectif d'activité d'un ordinateur personnel d'entraînement porté par l'utilisateur.

9. Procédé de l'une des revendications 1 à 8, comprenant en outre :
la fourniture, par le biais de l'interface et lors de la détection d'un événement prédéterminé, d'une instruction permettant à l'utilisateur de mesurer son poids.

10. Appareil comprenant :
des moyens pour acquérir au moins une valeur de poids représentant le poids d'un utilisateur ;
des moyens pour acquérir un objectif d'évolution de poids pour l'utilisateur ;
des moyens pour acquérir des données de mesure d'activité représentant une activité physique mesurée chez l'utilisateur dans un intervalle d'observation ;
des moyens pour acquérir des informations d'apport nutritionnel de l'utilisateur dans l'intervalle d'observation ;
des moyens pour calculer, en se basant sur l'au moins une valeur de poids et l'objectif d'évolution de poids, au moins une instruction d'ajustement d'activité indiquant une activité physique requise de l'utilisateur ; et
des moyens pour délivrer l'instruction d'ajustement d'activité par le biais d'une interface de l'appareil,
des moyens pour calculer, en se basant sur l'au moins une valeur de poids et de l'objectif d'évolution de poids, au moins une instruction d'ajustement d'apport nutritionnel indiquant un apport nutritionnel requis de l'utilisateur ; et
des moyens pour délivrer l'instruction d'ajustement d'apport nutritionnel par le biais de l'interface,
**caractérisé en ce que** l'appareil comprend en outre :
des moyens pour estimer, en se basant sur l'au moins une valeur de poids, une dépense énergétique calculée à partir des données de mesure d'activité et un apport énergétique calculé à partir des informations d'apport nutritionnel, une évolution future de poids de l'utilisateur ; et
des moyens pour délivrer des données représentant l'évolution future de poids par le biais de l'interface ; et
des moyens pour calculer l'au moins une instruction d'ajustement d'activité et l'au moins une instruction d'ajustement d'apport nutritionnel en se basant en outre sur l'évolution future de poids estimée, dans lequel l'au moins une instruction d'ajustement d'activité indique comment modifier l'activité physique par rapport à l'activité physique mesurée et l'au moins une instruction d'ajustement d'apport nutritionnel indique comment modifier l'apport nutritionnel par rapport aux informations d'apport nutritionnel de l'utilisateur.

11. Appareil de la revendication 10, comprenant en outre des moyens pour effectuer toutes les étapes du procédé selon l'une des revendications précédentes 2 à 9.

12. Produit de programme informatique incorporé dans un support de distribution lisible par un ordinateur et comprenant des instructions de programme qui, lorsqu'elles sont exécutées sur un ordinateur, exécutent le procédé selon l'une des revendications précédentes 1 à 9.
